# EUROPÄISCHE PATENTSCHRIFT

(11) **EP 1 658 106 B1**
(45) Veröffentlichungstag und Bekanntmachung des Hinweises auf die Patenterteilung: **20.09.2017**
(21) Anmeldenummer: 04737422.8
(22) Anmeldetag: 27.08.2004
(51) Int. Cl.: A61M 5/20

(54) **VORRICHTUNG ZUM AUTOMATISCHEN INJIZIEREN VON INJEKTIONSFLÜSSIGKEITEN**
DEVICE FOR AUTOMATICALLY INJECTING LIQUIDS TO BE INJECTED
DISPOSITIF D'INJECTION AUTOMATIQUE DE LIQUIDES A INJECTER

(30) Priorität: 29.08.2003 AT 59303 U
(43) Veröffentlichungstag der Anmeldung: 24.05.2006
(73) Patentinhaber: Pharma Consult Ges.m.b.H. & Co Nfg KG, 1210 Wien (AT)
(72) Erfinder: PICKHARD, Ewald, 2203 Grossebersdorf (AT)
(74) Vertreter: Walker, Ross Thomson
(86) Internationale Anmeldenummer: PCT/AT2004/000295
(87) Internationale Veröffentlichungsnummer: WO 2005/021070

(56) Entgegenhaltungen:
- WO-A-01/07104
- US-A- 3 742 948
- US-A- 4 624 660
- US-A- 5 709 668

## Beschreibung

Die Erfindung betrifft eine Vorrichtung zum automatischen Injizieren von Injektionsflüssigkeiten mit einem in axialer Richtung unterteilten Gehäuse, dessen Teile miteinander verbindbar sind, wobei in einem ersten Gehäuseteil ein axial verschieblicher Druckbolzen geführt ist, welcher gegen einen Kraftspeicher einschiebbar und in der eingeschobenen Lage verriegelbar ist und unter Entlastung des Kraftspeichers ausfahrbar ist und in einem zweiten Gehäuseteil eine in einer Kanülenführung festsitzende Injektionskanüle und eine Karpule in axialer Richtung relativ zueinander verschiebbar gelagert sind, wobei die Injektionskanüle an ihrer der Karpule zugewandten Seite als Durchstichstück für die Karpule ausgebildet ist.

Einrichtungen der eingangs genannten Art sind unter der Bezeichnung Autoinjektor bekannt geworden. Bei den bekannten Einrichtungen handelt es sich um Instrumente, welche bei Eintreten einer Notsituation das Injizieren eines Notfallmittels in den Körper erleichtern. Autoinjektoren werden beispielsweise bei Allergienotfällen, z.B. bei Insektenstichen, Schlangenbissen usw., angewendet aber auch im Militärbereich um beispielsweise Vergiftungen durch C-Kampfstoffe rasch entgegenzuwirken. Die bekannten Einrichtungen sind meist als Einwegeinrichtungen konzipiert und werden daher nach einmaliger Verwendung entsorgt.

Aus der AT 303 251 ist eine Injektionsvorrichtung bekannt geworden, welche aus zwei ineinander verschraubbaren Gehäuseteilen, den einen federbelastbaren Druckbolzen enthaltenden Aktivator und den die Karpule und die Injektionsnadel als untrennbare Einheit enthaltenden Injektor, besteht. Nach Entriegeln des federbelasteten Druckbolzens übt dieser eine Kraft auf den Kolbenstopfen der Karpule aus, worauf zunächst die Karpule gemeinsam mit der Injektionsnadel innerhalb des Injektorgehäuses in axialer Richtung verschoben wird, sodass die Injektionsnadel in den Körper des Patienten eindringt und in der Folge die in der Karpule befindliche Flüssigkeit unter einen so hohen Druck gelangt, dass eine zwischen Karpule und Injektionsnadel vorhandene Abdichtmembran bricht und dadurch die Flüssigkeit ausgestoßen wird. Nachteilig an dieser bekannten Ausbildung ist jedoch, dass die aufgebrochene Membran die Injektionsnadel verlegen kann, wodurch ein rasches Ausstoßen der Injektionsflüssigkeit verhindert wird.

Aus der WO 01/07104 ist bereits eine verbesserte Injektionsvorrichtung bekannt geworden, bei welcher die Karpule und die Injektionsnadel relativ zueinander in axialer Richtung verschiebbar im Gehäuse angeordnet sind und das der Karpule zugewandte Ende der Injektionskanüle als Durchstichstück für die Karpule ausgebildet ist. Dabei wird die Karpule im Inneren des Gehäuses gelagert ohne fest mit der Injektionskanüle verbunden zu sein. Erst im Falle der Anwendung wird die Karpule unter der Kraft des durch Entlastung des Kraftspeichers ausfahrenden Druckbolzens in Richtung der Injektionskanüle verschoben und von dem als Durchstichstück ausgebildeten Ende der Injektionskanüle aufgestochen.

Eine weitere Injektionsvorrichtung ist aus der US 4,624,660 bekannt.

Die vorbekannten Autoinjektoren können jedoch die aktuellen Anforderungen, wie sie für den Einsatz im militärischen Bereich vorgeschrieben werden, nur unzureichend erfüllen. Autoinjektoren, welche für militärische Zwecke geeignet sein sollen, müssen nämlich eine überdurchschnittlich lange Haltbarkeit und Sterilität der entsprechenden Injektionsvorrichtungen sicherstellen und überaus robust ausgeführt sein. Beispielsweise werden Autoinjektoren für militärische Zwecke probeweise aus einer Höhe von 2 Metern auf einen Steinboden fallengelassen, um zu überprüfen ob die Karpule bricht oder ob durch den mechanischen Impuls ein Verrutschen der Karpule und/ oder der Injektionskanüle derart erfolgt, dass die Injektionskanüle die Dichtscheibe der Karpule durchstößt. Ähnliche Tests werden auch unter extremen Temperaturbedingungen durgeführt.

Die Erfindung zielt nun darauf ab, eine Vorrichtung der eingangs genannten Art zu schaffen die den oben genannten Anforderungen für eine Verwendung im militärischen Bereich ausreichend Rechnung trägt. Zur Lösung dieser Aufgabe besteht die erfindungsgemäße Vorrichtung im Wesentlichen darin, dass die Karpule mit ihrem der Injektionskanüle zugewandten Ende in eine im zweiten Gehäuseteil festsitzende Hülse eintauchend gelagert ist, deren Innendurchmesser im wesentlichen dem Außendurchmesser der Karpule entspricht, dass am Innenumfang der Hülse radial einwärts ragende Vorsprünge ausgebildet sind, und dass die Hülse mit Verriegelungsgliedern der Kanülenführung zusammenwirkende Verriegelungsglieder aufweist, wobei eine axiale Verschiebung der Karpule in Richtung zur Kanülenführung unter Überwindung des von den Vorsprüngen ausgeübten Verschiebewiderstands die Freigabe der Verriegelungsglieder und der axialen Verschiebbarkeit der Kanülenführung bewirkt, wobei die verriegelungsglieder der Hülse an auswärts federnd auslenkbaren Armen ausgebildet sind, wobei die Arme im Bereich ihrer Anlenkstelle einen einwärtsragenden Vorsprung tragen, welcher mit der Karpule unter Auslenkung der Arme und Freigabe der Verriegelungsglieder zusammenwirkt. Dadurch, dass nun die Karpule mit ihrem der Injektionskanüle zugewandten Ende in eine im zweiten Gehäuseteil festsitzende Hülse eintauchend gelagert ist, können die am Innenumfang der Hülse angebrachten radial einwärts ragenden Vorsprünge, welche einen kleineren Durchmesser als den der Karpule definieren, ein Verrutschen der Karpule in Richtung zur Injektionskanüle verhindern. Äußerstenfalls wird die von einem Aufprall oder sonstiger mechanischer Einwirkung freiwerdende Energie von den einwärts ragenden Vorsprüngen unter Aufdehnung der Hülsenwand absorbiert. Dadurch bleibt die Karpule nach kurzer Verschiebung zwischen den einwärts ragenden Vorsprüngen stecken, wobei eine weitere Verschiebung verhindert wird.

Erfindungsgemäß ist die Hülse mit Verriegelungsgliedern ausgestattet, welche mit entsprechenden Verriegelungsgliedern der Kanülenführung zusammenwirken, sodass auch die Kanülenführung samt der darin festsitzenden Injektionskanüle gegen axiale Verschiebung gesichert wird. Sowohl die in die Hülse eintauchende Karpule als auch die mit der Hülse verbundene Kanülenführung sind somit an die Hülse gekoppelt, wobei die Hülse ihrerseits in dem zweiten Gehäuseteil unbeweglich gehalten ist, zu welchem Zweck bevorzugt die der Kanülenführung zugewandte stirnseitige Ringfläche der Hülse auf einem radial einwärts ragenden Vorsprung des zweiten Gehäuseteils aufliegt. Dadurch ist selbst beim Fall der Injektionsvorrichtung aus extremer Höhe auf harten Untergrund sichergestellt, dass sich die Karpule oder die Kanülenführung nicht lösen kann und die Injektionskanüle nicht aus dem Injektorgehäuse austreten kann.

Zur Aktivierung der Injektionsvorrichtung ist erfindungsgemäß vorgesehen, dass eine axiale Verschiebung der Karpule in Richtung zur Kanülenführung unter Überwindung des von den Vorsprüngen ausgeübten Verschiebewiderstands die Freigabe der Verriegelungsglieder und der axialen Verschiebbarkeit der Kanülenführung bewirkt. Die Verriegelungsglieder werden hierbei lediglich dann freigegeben, wenn die Karpule im Injektorgehäuse durch die Federkraft des entriegelten Druckbolzens in Richtung zur Injektionskanüle gestoßen wird, wobei derart hohe Kräfte freigesetzt werden, dass der von den Vorsprüngen der Hülse auf die Karpule ausgeübte Verschiebewiderstand überwunden wird. Die Freigabe der die Kanülenführung fixierenden Verriegelungsglieder erfolgt hierbei derart, dass die Verriegelungsglieder der Hülse an auswärts federnd auslenkbaren Armen ausgebildet sind, wobei die Arme im Bereich ihrer Anlenkstelle einen einwärts ragenden Vorsprung tragen, welcher mit der Karpule unter Auslenkung der Arme und Freigabe der Verriegelungsglieder zusammenwirkt. Dabei können die Verriegelungsglieder von in Aufnahmeöffnungen eingreifenden Rastnasen gebildet sein. Eine axiale Verschiebung der Karpule bewirkt somit ein Auflaufen der Karpule auf einwärts ragende vorsprünge, welche an auswärts federnden Armen ausgebildet sind, sodass eine weitere axiale Verschiebung ein Auslenken der Arme bewirkt und die an den Armen ausgebildeten Verriegelungsglieder außer Eingriff gelangen und die Kanülenführung freigeben.

Nach Freigabe der Kanülenführung kann die Karpule ungehindert durch die Hülse hindurchtreten und sich dem als Durchstichstück ausgebildeten Ende der Injektionskanüle nähern. Um den in der Folge zu erwartenden harten Aufprall der Karpule auf die Injektionskanüle abzdämpfen ist bevorzugt zwischen der Kanülenführung und der Karpule ein in axialer Richtung wirksames Federelement angeordnet. Durch die Federwirkung wird die Aufschlagsenergie absorbiert und es kann ein möglicher Bruch der Glaskarpule verhindert werden. Dabei kann das Federelement als in axialer Richtung kompremierbarer Federkorb einstückig mit der Kanülenführung ausgebildet sein, sodass der Federkorb zusätzlich als Distanzierung genutzt werden kann, um ein Aufschlagen der Karpule auf den Boden der Kanülenführung zu verhindern. Die Karpule wird hierdurch knapp vor dem Boden der Kanülenführung ihre Endposition erreichen und ein Bruch des Karpulenkopfes kann sicher verhindert werden.

Um ein frühzeitiges Austreten der Injektionsflüssigkeit zu verhindern ist die Ausbildung mit Vorteil derart weitergebildet, dass die Injektionskanüle in axialem Abstand von ihrem als Durchstichstück für die Karpule ausgebildeten Ende eine radiale Durchtrittsöffnung aufweist. Der Kanal der Injektionskanüle tritt somit nicht unmittelbar nach Aufstechen der Karpule in leitende Verbindung mit der Injektionsflüssigkeit, sondern erst nach einer weiteren axialen Verschiebung, bei welcher die radiale Durchtrittsöffnung der Injektionskanüle in die Karpule eintaucht. Um sicherzustellen, dass die Injektionsflüssigkeit über die radiale Durchtrittsöffnung in die Injektionskanüle gedrückt wird und nicht um die Injektionskanüle herum seitlich aus der durchstochenen Gummidichtscheibe des Karpulenkopfes austritt, ist die Ausbildung bevorzugt derart weitergebildet, dass die radiale Durchtrittsöffnung in axialer Richtung zwischen dem als Durchstichstück ausgebildeten Ende der Injektionskanüle und einem an der Kanülenführung angeordneten, die Injektionskanüle umgebenden ringförmigen Steg angeordnet ist, wobei der ringförmige Steg einen geschlossenen Ringraum zwischen dem Steg und der in die Karpule eintauchenden Injektionskanüle ausbildet.

Um die Bruchsicherheit der zumeist aus Glas bestehenden Karpule zu erhöhen ist die Ausbildung bevorzugt derart weitergebildet, dass die Karpule mit ihrem der Injektionskanüle abgewandten Ende in eine hülsenförmige Karpulenaufnahme eintauchend angeordnet ist, welche an ihrem Innenumfang eine Mehrzahl von in Längsrichtung verlaufenden lamellenartige Führungsrippen aufweist. Dabei wird die Karpule in der hülsenförmigen Karpulenaufnahme mittig zentriert positioniert. Die elastischen Führungsrippen der Karpulenaufnahme schützen die Glaskarpule vor Bruch, da die von außen auf die Injektionsvorrichtung einwirkenden Erschütterungen von den Führungsrippen großteils absorbiert und daher nicht an die Karpule übertragen werden. So ist sichergestellt, dass die Glaskarpule selbst bei starken Erschütterungen bruchsicher gelagert ist.

Um die geforderte Sterilität sicherzustellen ist mit Vorteil die dem den Druckbolzen aufweisenden ersten Gehäuseteil zugewandte Öffnung der Karpulenaufnahem durch eine gasdurchlässige Dichtfolie verschlossen. Durch diese gasdurchlässige Dichtfolie kann der Innenraum des komplet bestückten, beidseitig verschlossenen Injektionsteils mittels eines Gases sterilisiert werden, sodass die Keimfreiheit des Injektionsteiles über Jahre hinweg garantiert werden kann. Zur vollständigen Abdichtung kann zwischen dem Außenumfang der Karpulenaufnahme und dem Innenumfang des zweiten Gehäuseteils eine Dichtung, insbesondere ein O-Ring, angeordnet sein.

Die Erfindung wird nachfolgend anhand eines in der Zeichnung schematisch dargestellten Ausführungsbeispieles näher erläutert. In dieser zeigt Fig.1 einen Längsschnitt durch die erfindungsgemäße Vorrichtung, Fig.2 eine vergrößerte Schnittdarstellung des Aktivatorteils, Fig.3 eine vergrößerte Darstellung des Injektorteils, Fig.4 eine Teildarstellung der Injektionseinheit teilweise im Schnitt, Fig.5 eine auseinandergezogene Darstellung des Injektorteils, Fig.6 eine abgewandelte Ausbildung des Injektorteils und Fig.7a bis 7h den Funktionsablauf der erfindungsgemäßen Vorrichtung.

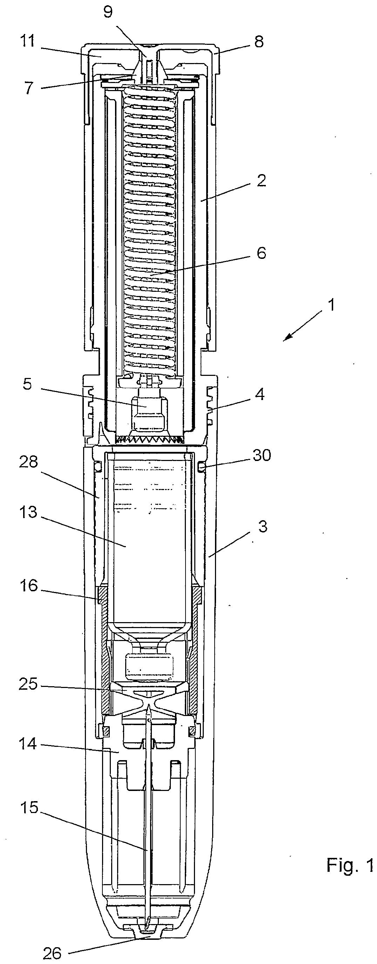

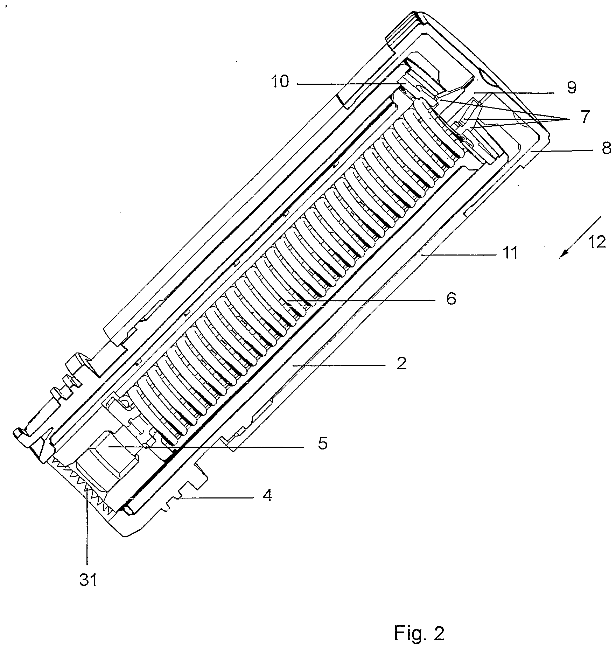

In Fig.1 ist die Injektionsvorrichtung 1 dargestellt deren Gehäuse in axialer Richtung unterteilt ist. Der erste Gehäuseteil 2 umschließt den Aktivatorteil, der zweite Gehäuseteil 3 umschließt den Injektionsteil der erfindungsgemäßen Vorrichtung, wobei beide Gehäuseteile mittels eines Gewindes 4 verbindbar sind. Der Aktivatorteil, welcher genauer in Fig.2 dargestellt ist, umfaßt einen im Gehäuse 2 axial verschieblichen Druckbolzen 5, welcher gegen eine Feder 6 einschiebbar ist. Der Druckbolzen 5 weist nach innen federnde Rastfortsätze 7 auf, welche den kegelförmigen Abschluß des Druckbolzens 5 bilden. In der verriegelten Lage hält ein Stift 9 einer Kappe 8 die Rastfortsätze 7 in einer radial nach außen ausgelenkten Position, sodass die Rastfortsätze 7 den ringförmigen Teil 10 übergreifen. Nach Abziehen der Kappe 8 kann der Aktivatorteil ausgelöst werden, indem die Sicherungshülse 11 in Richtung des Pfeiles 12 betätigt wird, wodurch die Rastfortsätze 7 nach innen gedrückt werden und der Druckbolzen 5 durch die Kraft der Feder 6 in Richtung des Pfeiles 12 katapultiert wird, wobei das von den Rastfortsätzen 7 gebildete kegelförmige Ende durch den lichten Querschnitt des Rings 10 hindurch tritt.

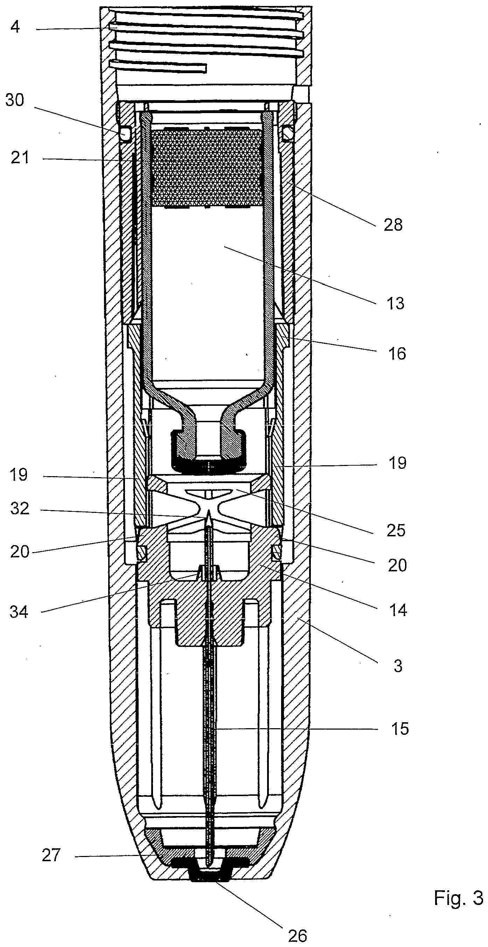

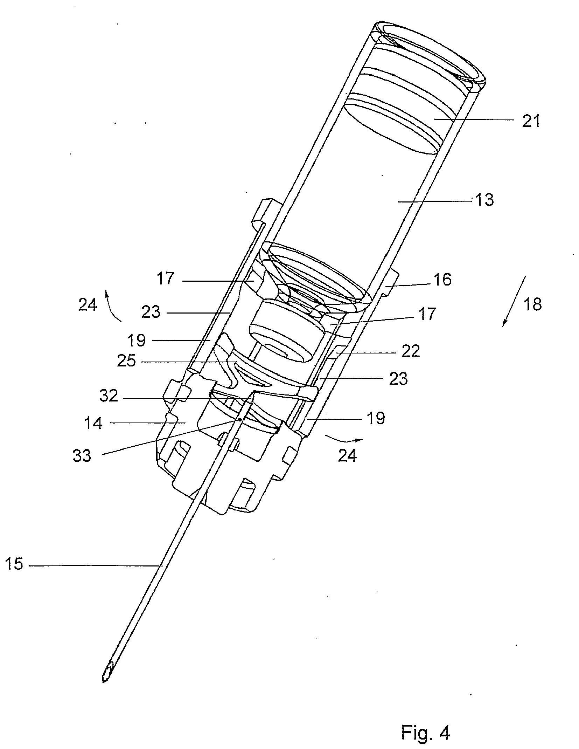

In Fig.3 ist der Injektorteil dargestellt, in welchem eine Karpule 13 und eine in einer Kanülenführung 14 festsitzende Injektionskanüle 15 gelagert sind. Die Karpule 13 taucht hierbei in eine Hülse 16 ein, welche ihrerseits im Gehäuse 3 gegen axiale Verschiebung gesichert ist. Wie aus Fig.4 ersichtlich ist, weist die Hülse 16 an ihrem Innenumfang radial einwärts vorragende Vorsprünge 17 auf, welche einen kleineren Durchmesser definieren als der Außendurchmesser der Karpule 13. Diese sind derart dimensioniert, dass die Karpule 13 auf den keilförmigen Auflaufflächen der Vorsprünge 17 aufliegt. Erst im Fall einer starken mechanischen Einwirkung auf den Injektorteil, beispielsweise dann, wenn dieser aus großer Höhe fallengelassen wird, erfolgt eine axiale Verschiebung der Karpule 13 in Richtung des Pfeiles 18, wobei die Karpule 13 entlang der keilförmigen Auflaufflächen der Vorsprünge 17 unter Aufdehnung der Hülse 16 in axialer Richtung weiter verschoben wird und in der Folge zwischen den Vorsprüngen 17 eingeklemmt wird und gegen weitere axiale Verschiebung gesichert ist.

Die Hülse 16 weist radial auswärts federnde Arme 19 auf, welche mit Ausnehmungen versehen sind, in welche entsprechende Rastnasen 20 der Kanülenführung 14 eintauchen. Dadurch ist die Kanülenführung 14 an die Hülse 16 gekoppelt und ebenfalls gegen axiale Verschiebung gesichert. Erst bei einer Betätigung der Injektionseinrichtung durch Auslösen des Druckbolzens 5 erfolgt eine Freigabe der Kanülenführung. Die Feder 6 des Aktivatorteils ist hierbei derart ausgelegt, dass der Druckbolzen 5 eine Kraft auf den Kolbenstopfen 21 ausübt, welche eine weitere Verschiebung der Karpule 13 in Richtung des Pfeiles 18 entgegen des durch die Vorsprünge 17 hervorgerufenen Verschiebewiderstands bewirkt. Die Karpule 13 trifft in der Folge über keilförmige Auflaufflächen 22 auf Vorsprünge 23 auf, welche an den federnden Armen 19 der Hülse 16 ausgebildet sind. Dies bewirkt eine Auslenkung der federnden Arme 19 in Richtung der Pfeile 24, sodass die Ausnehmungen nicht mehr von den Rastnasen 20 hintergriffen werden und die Kanülenführung 14 freigegeben werden.

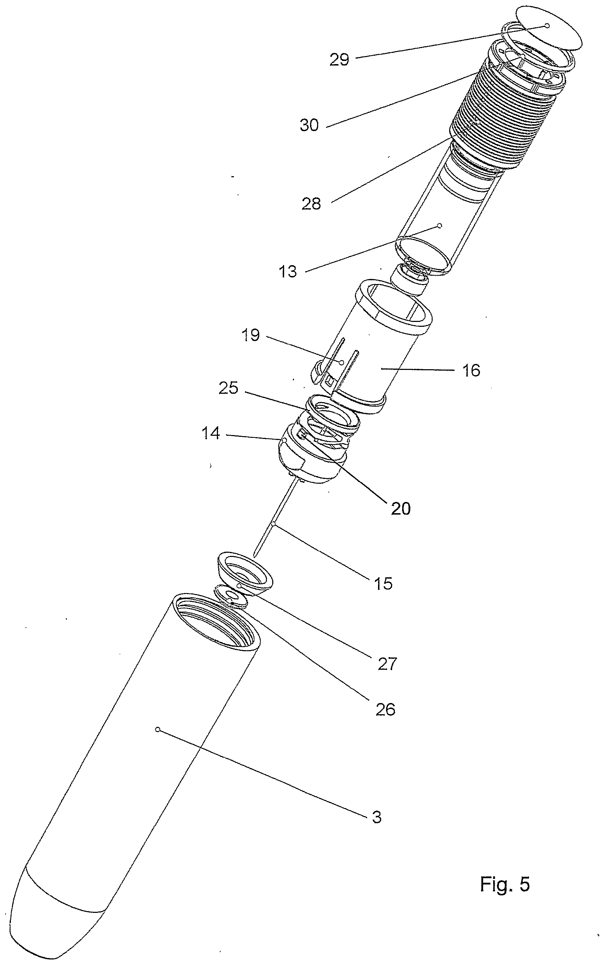

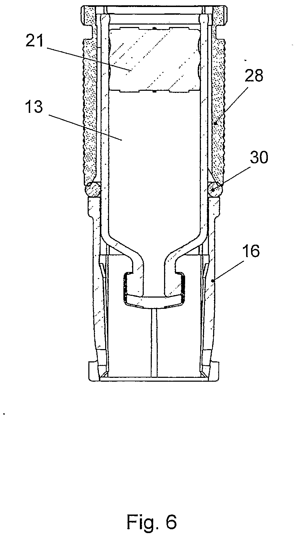

Um den Aufprall der Karpule 13 auf die Kanülenführung 14 zu dämpfen ist ein Federelement, und insbesondere ein Federkorb 25 vorgesehen, welcher einstückig mit der Kanülenführung 14 ausgebildet ist. In der Folge kann die Karpule 13 gemeinsam mit der Kanülenführung 14 in axialer Richtung weiterbewegt werden und die Injektionskanüle aus dem Gehäuse 3 austreten. In Fig.3 ist ersichtlich, dass das Gehäuse 3 von einer Nadelschutzkappe 26 verschlossen wird, welche in einem Klemmring 27 festgehalten ist. In Fig.3 ist weiters ersichtlich, dass die Karpule 13 in einer Karpulenaufnahme 28 gehalten ist, welche die Karpule 13 vor einem Bruch schützt. In der auseinandergezogenen Darstellung gemäß Fig.5 sind die einzelnen Teile des Injektorteils übersichtlich dargestellt und es ist auch ersichtlich, dass die Karpulenaufnahem 28 mittels einer Dichtfolie 29 verschlossen ist und gegen die Innenseite des Gehäuses 3 mit einem Dichtungsring 30 abgedichtet ist. Auf diese Art und Weise ist der gesamte Innenraum des Injektorteils gekapselt, sodass eine andauernde Sterilität der Injektionseinheit mit Karpule gewährleistet ist. Die Abdichtung des Injektorteilinnenraums kann jedoch auch auf andere Weise vorgenommen werden, wie in Fig.6 dargestellt. Die Karpulenaufnahme 28 weist an ihrem Außenumfang eine Labyrinthdichtung auf, welche eine Abdichtung der Karpulenaufnahme gegenüber dem Gehäuse 3 sicherstellt. Die Labyrinthdichtung weist hierbei eine Vielzahl von in Umfangsrichtung verlaufenden Lamellen auf, welche an einer Stelle entlang ihres Umfangs geschlitzt sind, wobei die Schlitze von in axialer Richtung benachbarten Lamellen um jeweils 180° versetzt sind. Das dabei entsehende Labyrinth ist für ein der Sterilisierung des Injektorteils dienendes Gas durchlässig, sodass dieses durch die Labyrinthdichtung hindurch in den Injektorteil eingeleitet werden kann. Die Labyrinthdichtung ist jedoch nicht für Bakterien durchlässig. Weiters ist der Dichtungsring 30 ersichtlich, welcher jedoch im Unterschied zur Ausbildung gemäß Fig.5 nicht zwischen Karpulenaufnahme 28 und Innenumfang des Gehäuses 3 sondern zwischen der Karpulenaufnahme 28 und der Karpule 13 angeordnet ist. Bei dieser Ausbildung ist eine gesonderte Dichtscheibe nicht mehr notwendig.

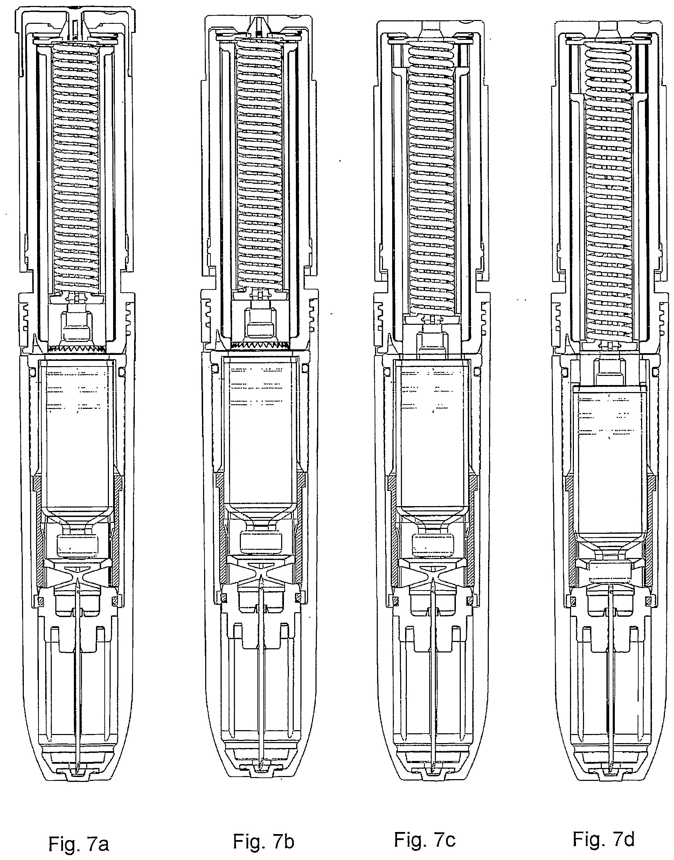

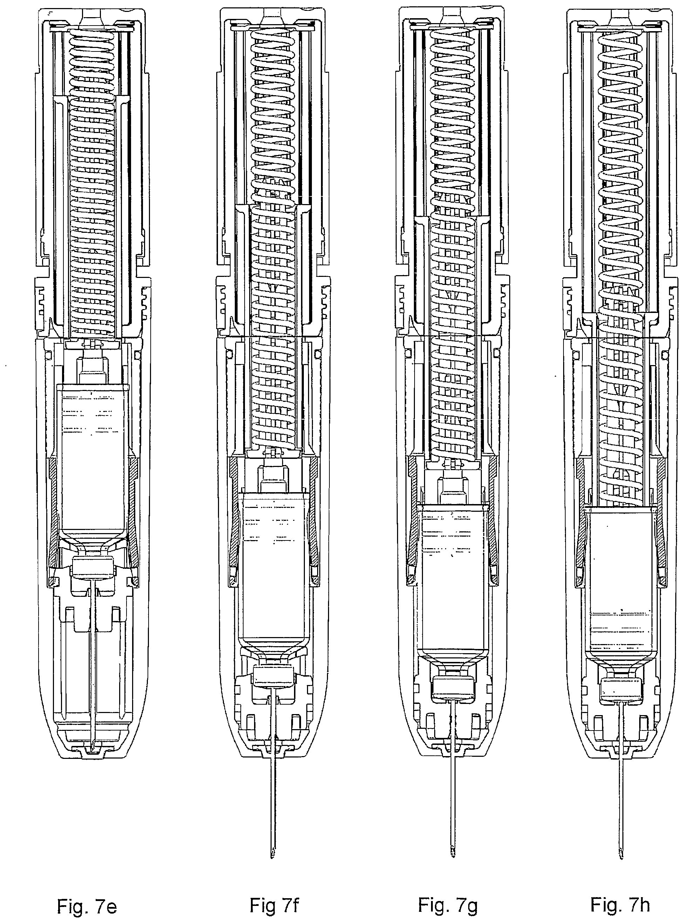

In den Fig. 7a bis 7h ist der Funktionsablauf beim Betätigen der Injektionsvorrichtung dargestellt. Fig.7a zeigt die Ausgangsposition vor dem Auslösen des Aktivators. In Fig.7b ist die Sicherungskappe 8 bereits entfernt worden und der Aktivator wird ausgelöst, wobei die Rastfortsätze 7 zusammengedrückt werden und den Druckbolzen 5 freigeben. In Fig.7c fährt der Druckbolzen 5 aus, wobei das mit einem Zahnkranz 31 versehene Ende des Druckbolzens 5 die Dichtfolie 29 durchstanzt. In Fig.7d wird die Karpule 13 entgegen des von den Vorsprüngen 17 ausgeübten Verschiebewiderstandes in axialer Richtung vorwärts geschoben und läuft auf die Vorsprünge 23 der federnden Arme 19 auf. In Fig.7e werden die beiden Federarme 19 der Hülse 16 auswärts verschwenkt und geben die Kanülenführung 14 frei. In Fig.7f durchsticht das karpulenseitige, als Durchstichstück 32 ausgebildete Ende der Injektionskanüle 15 die Nadelschutzkappe der Karpule 13. Die Karpule wurde hierbei gemeinsam mit der Kanülenführung 14 bis an die Endposition vorwärts bewegt, wobei die Injektionskanüle 15 in voller Länge aus dem Injektionsteil austritt. In Fig.7g wird unter weiterer Verschiebung der Karpule 13 der Federkorb 25 zusammengedrückt, sodass die Injektionskanüle 15 vollständig durch die Dichtscheibe der Karpule 13 hindurch tritt und die radiale Öffnung 33 der Injektionskanüle 15 in Verbindung mit der in der Karpule 13 enthaltene Injektionsflüssigkeit tritt. Gleichzeitig dringt der in Fig.3 dargestellte ringförmige Steg 34 in die Dichtscheibe der Karpule 13 ein und es bildet sich zwischen dem ringförmigen Steg 34 und der in die Karpule 13 eintauchenden Injektionskanüle 15 ein geschlossener Ringraum aus, welcher verhindert, dass die Injektionsflüssigkeit seitlich außerhalb der Injektionskanüle 15 aus der Karpule 13 austritt. Dadurch, dass die Injektionsflüssigkeit nun über die radiale Öffnung 33 und die Injektionskanüle 15 austreten kann, wird der Kolbenstopfen 21 durch den Druckbolzen 5 bis auf den Anschalg vorwärts getrieben, sodass die Injektionsflüssigkeit vollständig ausgespritzt wird.

## Patentansprüche

1. Vorrichtung zum automatischen Injizieren von Injektionsflüssigkeiten mit einem in axialer Richtung unterteilten Gehäuse, dessen Teile miteinander verbindbar sind, wobei in einem ersten Gehäuseteil (2) ein axial verschieblicher Druckbolzen (5) geführt ist, welcher gegen einen Kraftspeicher (6) einschiebbar und in der eingeschobenen Lage verriegelbar ist und unter Entlastung des Kraftspeichers (6) ausfahrbar ist und in einem zweiten Gehäuseteil (3) eine in einer Kanülenführung (14) festsitzende Injektionskanüle (15) und eine Karpule (13) in axialer Richtung relativ zueinander verschiebbar gelagert sind, wobei die Injektionskanüle (15) an ihrer der Karpule (13) zugewandten Seite als Durchstichstück für die Karpule (13) ausgebildet ist, wobei der Druckbolzen (5) durch Beaufschlagung mit einer in dem Kraftspeicher (6) gespeicherten Kraft eine Kraft auf den Kolbenstopfen (21) der Karpule (13) ausüben kann, um eine Verschiebung der Karpule (13), ein Einstechen der Injektionskanüle (15) und eine Injektion zu bewirken, **dadurch gekennzeichnet, dass** die Karpule (13) mit ihrem der Injektionskanüle (15) zugewandten Ende in eine im zweiten Gehäuseteil (3) festsitzende Hülse (16) eintauchend gelagert ist, deren Innendurchmesser im wesentlichen dem Außendurchmesser der Karpule (13) entspricht, dass am Innenumfang der Hülse (16) radial einwärts ragende Vorsprünge (17) ausgebildet sind, und dass die Hülse (16) mit Verriegelungsgliedern (20) der Kanülenführung (14) zusammenwirkende Verriegelungsglieder aufweist, wobei eine axiale Verschiebung der Karpule (13) in Richtung zur Kanülenführung (14) unter Überwindung des von den Vorsprüngen (17) ausgeübten Verschiebewiderstands die Freigabe der Verriegelungsglieder (20) und der axialen Verschiebbarkeit der Kanülenführung (14) bewirkt, wobei
die Verriegelungsglieder der Hülse (16) an auswärts federnd auslenkbaren Armen (19) ausgebildet sind, wobei die Arme (19) im Bereich ihrer Anlenkstelle einen einwärtsragenden Vorsprung (23) tragen, welcher mit der Karpule (13) unter Auslenkung der Arme (19) und Freigabe der Verriegelungsglieder (20) zusammenwirkt.

2. Vorrichtung nach Anspruch 1, **dadurch gekennzeichnet, dass** die der Kanülenführung (14) zugewandte stirnseitige Ringfläche der Hülse (16) auf einem radial einwärts ragenden Vorsprung des zweiten Gehäuseteils (3) aufliegt.

3. Vorrichtung nach Anspruch 1 oder 2, **dadurch gekennzeichnet, dass** die Verriegelungsglieder von in Aufnahmeöffnungen eingreifenden Rastnasen (20) gebildet sind, wobei die Rastnasen (20) vorzugsweise an der Kanülenführung (14) ausgebildet sind.

4. Vorrichtung nach einem der Ansprüche 1 bis 3, **dadurch gekennzeichnet, dass** zwischen der Kanülenführung (14) und der Karpule (13) ein in axialer Richtung wirksames Federelement angeordnet ist.

5. Vorrichtung nach Anspruch 4, **dadurch gekennzeichnet, dass** das Federelement als in axialer Richtung komprimierbarer Federkorb (25) einstückig mit der Kanülenführung (14) ausgebildet ist.

6. Vorrichtung nach einem der Ansprüche 1 bis 5, **dadurch gekennzeichnet, dass** die Injektionskanüle (15) in axialem Abstand von ihrem als Durchstichstück (32) für die Karpule (13) ausgebildeten Ende eine radiale Durchtrittsöffnung (33) aufweist.

7. Vorrichtung nach Anspruch 6, **dadurch gekennzeichnet, dass** die radiale Durchtrittsöffnung (33) in axialer Richtung zwischen dem als Durchstichstück (32) ausgebildeten Ende der Injektionskanüle (15) und einem an der Kanülenführung (14) angeordneten, die Injektionskanüle (15) umgebenden ringförmigen Steg (34) angeordnet ist, wobei der ringförmige Steg (34) in an einer Dichtscheibe der Karpule (13) anliegenden Zustand einen geschlossenen Ringraum zwischen dem Steg (34) und der in die Karpule (13) eintauchenden Injektionskanüle (15) ausbildet.

8. Vorrichtung nach einem der Ansprüche 1 bis 7, **dadurch gekennzeichnet, dass** die Karpule (13) mit ihrem der Injektionskanüle (15) abgewandten Ende in eine hülsenförmige Karpulenaufnahme (28) eintauchend angeordnet ist, welche an ihrem Innenumfang eine Mehrzahl von in Längsrichtung verlaufenden lamellenartige Führungsrippen aufweist.

9. Vorrichtung nach Anspruch 7, **dadurch gekennzeichnet, dass** die dem den Druckbolzen (5) aufweisenden ersten Gehäuseteil (2) zugewandte Öffnung der Karpulenaufnahme (28) durch eine gasdurchlässige Dichtfolie (29) verschlossen ist.

10. Vorrichtung nach Anspruch 8 oder 9, **dadurch gekennzeichnet, dass** zwischen dem Außenumfang der Karpulenaufnahme (28) und dem Innenumfang des zweiten Gehäuseteils (3) eine Dichtung,insbesondere ein O-Ring (30), angeordnet ist.

11. Vorrichtung nach Anspruch 7, **dadurch gekennzeichnet, dass** der Außenumfang der Karpulenaufnahme (28) eine Labyrinthdichtung aufweist und dass zwischen der Karpulenaufnahme (28) und der Karpule (13) eine Dichtung, insbesondere ein O-Ring (30), angeordnet ist.

## Claims

1. Device for automatically injecting liquids to be injected with a housing that is subdivided in the axial direction, the parts of said housing being interconnectable, wherein an axially displaceable stud (5) is guided in a first part (2) of the housing, which can be pushed in against an energy storage mechanism (6), can be locked in the pushed in position, and can be moved out when the energy storage mechanism (6) is released, and an injection canula (15), blocked in a canula guiding mechanism (14), is mounted, with an ampule (13), in a second part (3) of the housing, in such a way that they can be displaced in relation to each other, wherein the injection canula (15) is embodied as a puncturing part for the ampule (13) on the side thereof facing the ampule (13), wherein the stud (5), under the imposition of a force stored in the energy storage mechanism (6), can exert a force onto the plunger head (21) of the ampule (13) in order to cause a displacement of the ampule (13), the piercing of the injection canula (15), and to cause an injection, **characterized in that** the ampule (13) with its end facing the injection ampule (15) is plunged into a sleeve (16) blocked in the second part (3) of the housing, the inner diameter of the sleeve essentially corresponding to the outer diameter of the ampule (13), that radially inwardly protruding projections (17) are formed on the inner circumference of the sleeve (16), and that the sleeve (16) comprises locking elements which interact with locking elements (20) of the canula guiding mechanism (14), wherein an axial displacement of the ampule (13) in the direction of the canula guiding mechanism (14), overcoming the displacement resistance exerted by the projections (17), causes the release of the locking elements (20) and the axial displaceability of the canula guiding mechanism (14), wherein the locking elements of the sleeve (16) are formed on arms which can be deflected outwards under spring force, wherein the arms (19) carry, in the region of their guide point, an inwards projecting projection (23) which interacts with the ampule (13) under the deflection of the arms (19) and the release of the locking elements (20).

2. Device according to claim 1, **characterized in that** the face-side annular surface of the sleeve (16), facing towards the ampule guide mechanism (14), lies on a radially inwards projecting projection of the second part (3) of the housing.

3. Device according to claim 1 or 2, **characterized in that** the locking elements are formed from latch noses (20) engaging in receiving openings, wherein the latch noses (20) are preferably formed on the canula guiding mechanism (14)

4. Device according to one of claims 1 to 3, **characterized in that** a spring element, taking effect in the axial direction, is arranged between the canula guide mechanism (14) and the ampule (13).

5. Device according to claim 4, **characterized in that** the spring element is configured as a spring basket (25), compressible in the axial direction, and as one piece with the canula guide element (14).

6. Device according to one of claims 1 to 5, **characterized in that** the injection canula (15) comprises a radial passage opening (33) at an axial distance interval from its end which is configured as a puncture element (32) for the ampule (13).

7. Device according to claim 6, **characterized in that** the radial passage opening (33) is arranged in the axial direction between the end of the injection canula (15), configured as puncture element (32), and an annular web (34) surrounding the injection canula (15) arranged at the canula guide element (14), wherein the annular web (34), in the state in which it is in contact with a sealing disk of the ampule (13), forms a closed annular space between the web (34) and the injection canula (15) plunging into the ampule (13).

8. Device according to one of claims 1 to 7, **characterized in that** the ampule (13) is arranged with its end facing away from the injection canula (15) plunging into a sleeve-shaped ampule receiver (28), which comprises on its inner circumference a plurality of lamellar guide ribs running in the longitudinal direction.

9. Device according to claim 7, **characterized in that** the opening of the ampule receiver (28) facing the first part (2) of the housing, which comprises the stud (5), is closed by a gas-permeable sealing film (29).

10. Device according to claim 8 or 9, **characterized in that** a seal, in particular an O-ring (30), is arranged between the outer circumference of the ampule receiver (28) and the inner circumference of the second part (3) of the housing.

11. Device according to claim 7, **characterized in that** the outer circumference of the ampule receiver (28) comprises a labyrinth seal, and that a seal, in particular an O-ring (30), is arranged between the ampule receiver (28) and the ampule (13).

## Revendications

1. Dispositif d'injection automatique de liquides à injecter avec un boîtier qui est sous-divisé dans le sens axial, les parties dudit boîtier étant interconnectables, dans lequel un goujon pouvant se déplacer axialement (5) est guidé dans une première partie (2) du boîtier, lequel peut être poussé contre un mécanisme de stockage d'énergie (6), peut être verrouillé en position poussée et peut être sorti lorsque le mécanisme de stockage d'énergie (6) est libéré, et une canule d'injection (15), bloquée dans un mécanisme de guidage de canule (14), est montée, avec une ampoule (13), dans une deuxième partie (3) du boîtier, de telle sorte qu'elles peuvent être déplacées l'une par rapport à l'autre, dans lequel la canule d'injection (15) se présente sous la forme d'une partie perforante de l'ampoule (13) du côté de celle-ci étant face à l'ampoule (13), dans lequel le goujon (5), sous l'imposition d'une force stockée dans le mécanisme de stockage d'énergie (6), peut exercer une force sur la tête de plongeur (21) de l'ampoule (13) afin de causer un déplacement de l'ampoule (13), le perçage de la canule d'injection (15), et de causer une injection, **caractérisé en ce que** l'ampoule (13) avec son extrémité tournée vers l'ampoule d'injection (15) est plongée dans une douille (16) bloquée dans la deuxième partie (3) du boîtier, le diamètre intérieur de la douille correspondant sensiblement au diamètre intérieur de l'ampoule (13), **en ce que** des saillies dépassant radialement vers l'intérieur (17) sont formées sur la circonférence intérieure de la douille (16), et **en ce que** la douille (16) comprend des éléments de verrouillage qui interagissent avec des éléments de verrouillage (20) du mécanisme de guidage de canule (14), dans lequel un déplacement axial de l'ampoule (13) dans le sens du mécanisme de guidage de canule (14), surmontant la résistance au déplacement exercée par les saillies (17), cause la libération des éléments de verrouillage (20) et le déplacement axial du mécanisme de guidage de canule (14), dans lequel les éléments de verrouillage de la douille (16) sont formés sur des bras (19) qui peuvent être fléchis vers l'extérieur sous la force de ressort, dans lequel les bras (19) portent, dans la région de leur point de guidage, une saillie dépassant vers l'intérieur (23) qui interagit avec l'ampoule (13) sous le fléchissement des bras (19) et la libération des éléments de verrouillage (20).

2. Dispositif selon la revendication 1, **caractérisé en ce que** la surface annulaire face à la douille (16), tournée vers le mécanisme de guidage de l'ampoule (14), se trouve sur une saillie dépassant radialement vers l'intérieur de la deuxième partie (3) du boîtier.

3. Dispositif selon la revendication 1 ou 2, **caractérisé en ce que** les éléments de verrouillage sont formés par des nez de verrouillage (20) s'engageant dans des ouvertures de réception, dans lequel les nez de verrouillage (20) sont de préférence formés sur le mécanisme de guidage de canule (14)

4. Dispositif selon l'une quelconque des revendications 1 à 3, **caractérisé en ce qu'**un élément de ressort, effectif dans le sens axial, est agencé entre le mécanisme de guidage de canule (14) et l'ampoule (13).

5. Dispositif selon la revendication 4, **caractérisé en ce que** l'élément de ressort est configuré sous la forme d'un panier ressort (25), compressible dans le sens axial, et d'un seul tenant avec l'élément de guidage de canule (14).

6. Dispositif selon l'une quelconque des revendications 1 à 5, **caractérisé en ce que** la canule d'injection (15) comprend une ouverture de passage radiale (33) à un intervalle de distance axial de son extrémité laquelle est configurée sous la forme d'un élément de perforation (32) de l'ampoule (13).

7. Dispositif selon la revendication 6, **caractérisé en ce que** l'ouverture de passage radiale (33) est agencée dans le sens axial entre l'extrémité de la canule d'injection (15), configurée sous la forme d'un élément de perforation (32) et une moulure annulaire (34) entourant la canule d'injection (15) agencée à l'élément de guidage de canule (14), dans lequel la moulure annulaire (34), dans l'état où elle est en contact avec un disque d'étanchéité de l'ampoule (13), forme un espace annulaire fermé entre la moulure (34) et la canule d'injection (15) plongeant dans l'ampoule (13).

8. Dispositif selon l'une quelconque des revendications 1 à 7, **caractérisé en ce que** l'ampoule (13) est agencée avec son extrémité à l'opposé de la canule d'injection (15) plongeant dans un récepteur d'ampoule en forme de douille (28), lequel comprend sur sa circonférence intérieure une pluralité de nervures de guidage lamellaires dans le sens longitudinal.

9. Dispositif selon la revendication 7, **caractérisé en ce que** l'ouverture du récepteur d'ampoule (28) face à la première partie (2) du boîtier, qui comprend le goujon (5), est fermée par un film d'étanchéité perméable aux gaz (29).

10. Dispositif selon la revendication 8 ou 9, **caractérisé en ce qu'**un joint d'étanchéité, en particulier un joint torique (30), est agencé entre la circonférence extérieure du récepteur d"ampoule (28) et la circonférence intérieure de la deuxième partie (3) du boîtier.

11. Dispositif selon la revendication 7, **caractérisé en ce que** la circonférence extérieure du récepteur d'ampoule (28) comprend un joint à labyrinthe, et **en ce qu'**un joint d'étanchéité, en particulier un joint torique (30), est agencé entre le récepteur d'ampoule (28) et l'ampoule (13).
